Europäisches Patentamt

(19) European Patent Office

Office européen des brevets

(11) EP 0 592 107 B1

(12) EUROPEAN PATENT SPECIFICATION

(45) Date of publication and mention
of the grant of the patent:
**30.06.1999 Bulletin 1999/26**

(51) Int. Cl.[6]: **A61K 7/035**

(21) Application number: **93307068.2**

(22) Date of filing: **08.09.1993**

(54) **Color cosmetic power**

Kosmetisches Farbpuder

Poudre cosmétique colorée

(84) Designated Contracting States:
**AT BE CH DE DK ES FR GB GR IE IT LI NL PT SE**

(30) Priority: **10.09.1992 US 943164**
**10.09.1992 US 943073**

(43) Date of publication of application:
**13.04.1994 Bulletin 1994/15**

(73) Proprietors:
• **UNILEVER PLC**
**London EC4P 4BQ (GB)**
Designated Contracting States:
**GB IE**
• **UNILEVER N.V.**
**3013 AL Rotterdam (NL)**
Designated Contracting States:
**BE CH DE DK ES FR GR IT LI NL PT SE AT**

(72) Inventors:
• **Elliott, Marianne**
**Seymour, Connecticut 06843 (US)**
• **Gallagher, Lee Ann**
**Hamden, Connecticut 06514 (US)**
• **Hechavarria, Caridad**
**New Haven, Connecticut 06513 (US)**

(74) Representative:
**Rots, Maria Johanna Francisca et al**
**Unilever PLC,**
**Patent Division,**
**Colworth House**
**Sharnbrook, Bedford MK44 1LQ (GB)**

(56) References cited:
EP-A- 0 135 060          EP-A- 0 447 287
EP-A- 0 479 387          US-A- 5 073 364

• PATENT ABSTRACTS OF JAPAN vol. 012, no.
037 (C-473)4 February 1988 & JP-A-62 187 405
(NONOGAWA SHOJI KK) 15 August 1987
• PATENT ABSTRACTS OF JAPAN vol. 010, no.
280 (C-374)24 September 1986 & JP-A-61 100
508 (SHISEIDO CO LTD) 19 May 1986

## Description

The invention relates to color cosmetic compositions in powder form; in particular it relates to powder color cosmetic compositions that may be used as eyeshadow formulations, or blush formulations.

Molded powder color cosmetics in major part consist of fillers and extenders. Most often they are of the mineral variety. Illustrative are talc, kaolin, mica and silicon dioxide. In more limited amount, the extenders or fillers may be organic polymers including nylon and polyethylene. Often one or more of the aforementioned substances are blended together. These blends utilize relative proportions determined from consideration of skinfeel, spreadability, opacifying effect, moldability and adherence.

Boron nitride is a recent addition to the arsenal of useful extenders or fillers for press-molded color cosmetic compositions. For instance, JP-A-62-49427 (Ohno et al) and JP-A-62-187405 (Obara et al) have reported that inclusion of boron nitride powder affords a product having good spreadability and adherence while being smooth, lustrous and of high covering power.

EP-A-447 287 discloses the use of hollow microspheres comprising boron nitride in the use of mixtures in the preparation of cosmetic compositions while the intermediate application WO-A-93/04668 discloses the use of a coupling agent together with an outer boron nitride coating of particles for use in cosmetic compositions.

Investigations into the use of boron nitride powder has, however, revealed certain problems with skinfeel and moldability. Improvements would, therefore, be desirable in boron nitride containing systems.

Accordingly it is an object of the present invention to provide a pressed powder color cosmetic composition which exhibits improved properties of skinfeel, spreadability, creaminess, adhesion to skin and smoothness.

A further object of the present invention is to provide a pressed powder cosmetic color composition with good compression in manufacture.

A still further object of the present invention is to provide a pressed powder cosmetic color composition that is neither too dusty nor brittle.

These and other objects of the present invention will become more apparent by consideration of the following summary, detailed description and examples.

Thus, according to one aspect of the invention, there is provided a powder color cosmetic composition suitable for topical application to the human skin comprising:

i) from 0.001 to 30% by weight of an ultrafine powdered boron nitride having a particle size of 1 to 10 micrometer;
ii) from 0.001 to 30% by weight of a cosmetic adjunct material comprising powdered nylon having an average particle size ranging from 2-6 micrometer; and
iii) from about 0.5 to about 99% by weight of a pharmaceutically acceptable vehicle.

Now it has been found that improved pressed powder color cosmetic compositions can be achieved which are based on ultrafine boron nitride. In particular, many of the advantageous properties attributable to compositions according to the invention may be due to the ultrafine nature of the boron nitride used in the compositions.

Improved spreadability, adherence, skinfeel and covering powder are achieved with this composition while still maintaining excellent manufacturing compressibility. Of particular improvement is the balance achieved between payoff (i.e. yield against brush stroke) and integrity of the pressed powder cake.

Improved spreadability, skin adherence, skin feel and covering power are achieved with the combination of boron nitride with powdered nylon while still maintaining excellent manufacturing compressibility. Of particular advantage is that the normal disadvantages of nylon (poor adherence, dustiness and brittleness) and disadvantages of boron nitride (softness and poor compression) can mutually be overcome to provide a product of unusual properties.

According to the present invention in its broadest sense there is required as a first component of the powder composition an ultrafine powdered boron nitride. A wide variety of boron nitrides may be employed including the h-BN, w-BN, c-BN, r-BN and t-BN varieties. Average particle size of the boron nitride based upon spherical morphology can range from 1 to 10 micrometer, preferably from 3 to 7 micrometer. Most preferred is a boron nitride with a D50 mean value of 7.5 to 8 microns.

Amounts of the boron nitride powder may range from 0.001 to 30%, preferably from 1 to 10%, optimally from 4 to 7% by weight.

In a preferred aspect of the invention, the nylon is nylon 12 in powdered form. Particularly preferred is nylon 12 which has an average particle size ranging from 1 to 10 micrometer, preferably from 2 to 6 micrometer.

For purposes of this invention, the relative amounts of nylon to boron nitride should range from 10:1 to 1:30, preferably from 5:1 to 1:20.

A further element of the cosmetic composition according to the present invention is that of a pharmaceutically acceptable vehicle. The vehicle may be chosen from talc, inorganic pigments, metal oxide and hydroxides, mica, pearling pigments organic pigments, mineral silicates, porous materials, carbons, metals, biopolymers and combinations

EP 0 592 107 B1

thereof.

An important vehicle for use with compositions of the present invention is that of talc. The talc may have hydrophobic or hydrophilic surfaces, the former being achieved through treatment with a silicone such as methicone. Amounts of the talc may range anywhere from about 1 to 95%, preferably between 30 and 70%, optimally between 40 and 60% by weight. Talc average particle size should range from 0.5 to 9 micrometer, optimally between 6 and 8 micrometer. Levels below 4 micrometer or above 9 micrometer are ordinarily unsatisfactory when combined with boron nitride powder.

Examples of inorganic pigments are ultramarine blue, Prussian blue, manganese violet and bismuth oxychloride.

Examples of metal oxides and hydroxides useful in the present invention are magnesium oxide, magnesium hydroxide, magnesium carbonate, calcium oxide, calcium hydroxides, aluminum oxide, aluminum hydroxide, silica, iron oxides ($\alpha$-Fe$_2$O$_3$, y-Fe$_2$O$_3$, Fe$_3$O$_4$, FeO), iron hydroxides, titanium dioxide, titanium lower oxides, zirconium oxides, chromium oxides, chronium hydroxides, manganese oxides, cobalt oxides, nickel oxides and zinc oxides. These oxides and hydroxides may be used alone or in any mixture thereof. Furthermore, composite oxides and composite hydroxides such as iron titanate, cobalt titanate and cobalt aluminate can also be used in the present invention. Composite materials comprising metal oxides or hydroxides coated on the core materials (e.g. titanium oxides, coated mics, iron oxides coated nylon) also can be used in the present invention.

Examples of mica capable of being suitable for the present invention are muscovite, phlogopite, tiotite, sericite, lepidolite paragonite and artificial or synthetic mica having a fluorine atom substituted for the hydroxyl group of natural mica as well as baked or calcined products thereof. These mica may be used alone or in any mixture thereof. Particularly preferred is a hydrophobic mica wherein the mineral has been coated with a silicone compound such as cyclomethicone or dimethicone.

Examples of organic pigments suitable for the present invention are C.I. 15850, C.I. 15850:1, C.I. 15585:1, C.I. 15630, C.I. 15880:1, C.I. 73360, C.I. 12085, C.I. 15865:2, C.I. 12075, C.I. 21110, C.I. 21095, and C.I. 11680, C.I. 74160 and zirconium, barium or aluminium lakes of C.I. 45430, C.I. 45410, C.I. 45100, C.I. 17200, C.I. 45380, C.I. 45190, C.I. 14700, C.I. 15510, C.I. 19140, C.I. 15985, C.I. 45350, C.I. 47005, C.I. 42053 and C.I. 42090.

The surfaces of these organic pigments may be treated with, for example, resins. These organic pigments may be used alone or in any mixture thereof.

Examples of pearling pigments (or nacreous pigments) are bismuth oxychloride, guanine and titanium composite materials containing, as a titanium component, titanium dioxide, titanium lower oxides or titanium oxynitride. The titanium composite materials may be mixed with colored pigments such as iron oxides, Prussian blue, chromium oxide, carbon black and carmine. These pearling pigments may be used alone or in any mixture thereof.

Examples of mineral silicates suitable for the present invention are phyllosilicates and tectosilicates such as pyrophyllite, talc, chlorite, chrysotile antigorite, lizardite, kaolinite, dickite, nacrite, halloysite. montmorillonite, nontronite, saponite, sauconite, and bentonite; natrolites such as natrolite, mesolite, scolecite, and thomsonite; heulandites such as heulandite, stilbite, epistibite; and zeolites such as analcite harmotone, phillipsite, chabazite and gmelinite. These silicate minerals may be used alone or in combination thereof. The phyllosilicates may have organic cations at the interface of the layers thereof or may be substituted with alkali metal or alkaline earth metal ions. The tectosilicates may include metallic ions in the fine pores thereof.

Examples of porous materials suitable for the present invention are the above-mentioned silicate minerals; the above-mentioned mica; the above-mentioned metal oxides; KAl$_2$(Al Si$_3$)O$_{10}$F$_2$, KMg(Al, Si$_3$)O$_{10}$F$_2$, and K(Mg,Fe$_3$)(Al, Si$_3$)O$_{10}$F$_2$; carbonate minerals such as CaCO$_3$, MgCO$_3$, FeCO$_3$, MNCO$_3$, ZnCO$_3$, CaMg(CO$_3$)$_2$, Cu(OH)$_2$CO$_3$, and Cu$_3$(OH)$_2$(CO$_3$)$_2$ ; sulfate minerals such as BaSO$_4$, PbSO$_4$, CaSO$_4$, CaSO$_4$.2H$_2$O, CaSO$_4$.5(H$_2$O), Cu$_4$SO$_4$(OH)$_6$, KAl$_3$(OH)$_6$(SO$_4$)$_2$, and KFe$_3$(OH)$_6$(SO$_4$); phosphate minerals such as YPO$_4$, (CeLa)PO$_4$, Fe$_3$(PO$_4$)$_2$.8H$_2$O, Ca$_5$(PO$_4$)$_3$OH and Ca$_5$(PO$_4$CO$_3$OH)$_3$F, OH); and metal nitrides such as titanium nitride and chromium nitride. These materials may be used alone or in any mixture thereof.

Examples of metals suitable for the present invention are iron, cobalt, nickel, copper, zinc, aluminum, chromium, titanium, zirconium, molybdenum, silver, indium, tin, antimony, tungsten, platinum and gold, and the alloys thereof.

Powdery biopolymer materials are also suitable for the present invention, especially by virtue of their high safety factor.

Examples of biopolymer materials suitable for the present invention are keratin (hair, fur, feather, down, horn, hoof, etc.), fibroin (silk), collagen (skin, hide, leather, tendon, bond, etc.), cellulose, hemicellulose, pectin, chitin, chondroitin, peptide-glucan, nucleic acid (DNA, RNA) and the like.

Any conventional cosmetic ingredients can be used together with the powder materials. Typical examples of such ingredients are various hydrocarbons such as squalane, liquid paraffin, and microcrystalline wax; emollient acids and alcohols such as caprylic acid, myristic acid, palmitic acid, stearic acid, oleic acid, isostearic acid, cetyl alcohol, hexadecyl alcohol and oleyl alcohol; emollient esters such as caprylate esters, cetyl-2-ethylhexanoate, ethylhexyl palmitate, 2-octyldodecyl myristate, 2-octyldodecyl gum ester, neopentyl glycol-2-ethylhexanoate, isooctyl triglyceride, 2-octyldodecyl oleate, isopropyl myristate, isostearic acid triglycerides, coconut oil fatty acid triglyceride, olive oil, avocado oil, beeswax, myristyl myristate, mink oil, lanolin and dimethyl polysiloxane; resins such as alkyd resins and urea resins; plasticizers such as camphor and acetyl tributyl citrate; UV absorbers; antioxidants; preservatives; surfactants; humectants; perfumes; water; alcohols and thickening agents.

Particularly preferred additional components are the volatile silicone oils represented by dimethicone and cyclomethicone. These may be present in amounts ranging from 0.01 to about 10%, preferably between 1 and 5% by weight. Particularly preferred ester type ingredients are octyl palmitate and pentaerythritol tetra (2-ethyl hexanoate) and combinations thereof. The esters may be present in amounts from 0.1 to 20%, preferably between 1 and 5% by weight.

Among the preservatives useful for the present invention are phenoxyethanol, ethyl paraben, isobutyl paraben, n-butyl paraben, methyl paraben, propyl paraben, sodium dehydroacetate and combinations thereof. The amount of preservative may range from 0.01 to 5%, preferably between 0.10 and 2%, optimally between 0.4 and 1% by weight.

Various herbal extracts may also be employed. Examples of these extracts are rosemary, althea, sambucus, matricaria and combinations thereof. Levels of the extract may range from 0.0001 to 10%, preferably 0.1 to 2% by weight.

Compositions of the present invention advantageously will have all components of a similar average particle size, preferably between 1 and 8 micrometer, optimally between 2 and 7 micrometer. Uniformity of particle size may either be achieved by separately combining components of the proper size or by shear mixing the total composition down to the desired particle size range. A jet mill is particularly useful for purposes of shearing the total composition.

The following Example will more fully illustrate an embodiment of the present invention. All parts, percentages and proportions referred to herein and in the appended claims are by weight unless otherwise indicated.


EXAMPLE 1

An eye shadow formula according to the present invention is outlined under Table I


TABLE I

| Chemical or CTFA Name | Weight % |
| --- | --- |
| Talc/Methicone | QS |
| Mica/Methicone | QS |
| Silica Beads | 3.8-5 |
| Zinc Stearate | 0-6 |
| Nylon 12 | 2-4 |
| Boron Nitride | 5.00 |
| Methyl Paraben | 0.20 |
| Propyl Paraben | 0.10 |
| Sodium Dehydroacetate | 0.20 |
| Bismuth Oxychloride | 0-10 |
| Matricaria Extract | 0-1 |
| Rosemary Extract | 0-1 |
| Althea Extract | 0-1 |
| Sambucus Extract | 0-1 |
| Octyl palmitate | 0-5 |
| Pentaerythritol tetra (2-ethyl hexanoate) | 0-5 |
| Dimethicone | 0-5 |
| Colorants | 1-30 |


Colorants listed below will vary in percentages due to shade. The colorant can range from 1 to 30% depending upon the shade.

| Carmine | C.I. 75470 |
| Chromium Oxide, Hydrous Green | C.I. 77289 |
| Chromium Oxide, Anhydrous Green | C.I. 77288 |
| Ultramarine Rose | C.I. 77007 |
| Brown Iron Oxide | C.I. 77491, 2, 9 |
| Red Iron Oxide | C.I. 77491 |
| Russet Iron Oxide | C.I. 77491 |
| Yellow Iron oxide | C.I. 77492 |
| Black Iron Oxide | C.I. 77499 |
| Manganese Violet | C.I. 77742 |
| Ultramarine Blue | C.I. 7707 |
| Titanium Dioxide | C.I. 77891 |
| Prussian Blue | C.I. 77510 |
| Pearl Substrate: | Mica and/or $TiO_2$; Carmine; Iron Oxides; Ferric Ferrocyanide; Ultramarine Rose; Manganese Violet; Chromium Oxide Hydrous/Anhyrous Green; Ferrocyanide; Bismuth Oxychloride. |

A series of samples were formulated to evaluate properties of a pressed powder color cosmetic containing boron nitride and various particle size nylon 12 as the cosmetic adjunct. In these compositions, the binder was an approximately equal weight mixture of octyl palmitate, pentaerythritol tetra (2-ethyl hexanoate) and dimethicone. Table II lists the formulations.

TABLE II

| Component | Sample (Wt.%) | | | |
| --- | --- | --- | --- | --- |
| | A | B | C | D |
| Talc J68 | 32.5 | 32.5 | 32.5 | 32.5 |
| Hydrophobic Mica | 32.5 | 32.5 | 32.5 | 32.5 |
| Binder | 3.0 | 3.0 | 3.0 | 3.0 |
| Red Iron Oxide (50%) | 2.0 | 2.0 | 2.0 | 2.0 |
| Boron Nitride (3-7 micron) | 15.0 | 15.0 | 30.0 | - |
| Nylon (20-30 micron) | 15.0 | - | - | - |
| Nylon (2-6 micron) | - | 15.0 | - | 30.0 |
| | 100.0% | 100.0% | 100.0% | 100.0% |

Portions of each of the samples were filled into a cup of a press and compressed at 56.25 kg/cm$^2$ (800 psi) to a set fill line. The resultant cakes were of uniform volume and had a weight of approximately 3 grams each. These samples were then tested with a penetrometer. An acceptable cake will normally have a penetrometer reading from 7 to 15. Values below 7 indicate an unacceptable brick-like cake. Values greater than 15 reflect cakes that are too soft and provide too much payoff when rubbed with a brush. Each reported penetrometer reading in table III is the average of three determinations; mean deviations ranged from 0.9 to 1.5.

A second performance evaluation was that of the Drop Test. This test measures the firmness of a cake. Failure is noted if the cake cracks or, on the other end of the scale, if through softness, the cake is insufficiently adherent and falls out of its pan.

TABLE III

| Performance Properties | Sample | | | |
|---|---|---|---|---|
| | A | B | C | D |
| Penetrometer (mm) | 13.3 | 11.7 | 15.1 | 7.7 |
| Drop Test | Failed | Passed | Passed | Failed |

The results listed under Table VI indicate failure in the Drop Test for sample A wherein boron nitride is combined with a relatively large particle size nylon (20-30 micrometer). By contrast, Sample B that combines boron nitride with an ultrafine nylon of 6 micrometer average particle size passed the Drop Test. Sample C with boron nitride alone, in the absence of nylon, passed the Drop Test but went slightly beyond the acceptable range for a penetrometer value. Ultrafine nylon without boron nitride, as in Sample D, resulted in a Drop Test failure.

The foregoing description and examples illustrates selected embodiments of the present invention. In light thereof, various modifications will be suggested to one skilled in the art, all of which are within the spirit and purview of this invention.

**Claims**

1. A powdered colour cosmetic composition suitable for topical application to the human skin, comprising:

   (i) from 0.001 to 30% by weight of an ultrafine powdered boron nitride having a particle size of 1 to 10 $\mu$m;

   (ii) from 0.001 to 30% by weight of a cosmetic adjunct material comprising powdered nylon having an average particle size ranging from 2-6 $\mu$m;

   (iii) from 0.5 to 99% by weight of a pharmaceutically acceptable vehicle selected from the group consisting of talc, inorganic pigments, metal oxide and hydroxides, mica, pearling pigments, organic pigments, mineral silicates, porous materials, carbons, metals, biopolymers and combinations thereof.

2. A composition according to claim 1, wherein the nylon is nylon 12.

3. A composition according to claim 1 or 2, which is a blush formulation.

4. A composition according to claim 1 or claim 2, which is an eyeshadow formulation.

**Patentansprüche**

1. Eine kosmetische Farbpuder-Zusammensetzung, geeignet für die örtliche Anwendung auf die menschliche Haut, enthaltend:

   (I) Von 0,001 bis 30 Gewichtsprozent eines ultrafein gepulverten Bornitrids mit einer Teilchengröße von 1 bis 10 $\mu$m;

   (II) von 0,001 bis 30 Gewichtsprozent eines kosmetischen Zusatzmaterials, enthaltend pulverisiertes Nylon mit einer durchschnittlichen Teilchengröße im Bereich von 2 bis 6 $\mu$m;

   (III) von 0,5 bis 99 Gewichtsprozent eines pharmazeutisch annehmbaren Trägers, ausgewählt aus der Gruppe bestehend aus Talk, anorganischen Pigmenten, Metalloxiden und -hydroxiden, Glimmer, perlenden Pigmenten, organischen Pigmenten, Mineralsilicaten, porösen Materialien, Kohlen, Metallen, Biopolymeren, und Kombinationen derselben.

2. Eine Zusammensetzung nach Anspruch 1, worin das Nylon Nylon 12 ist.

3. Eine Zusammensetzung nach Anspruch 1 oder 2, welche eine errötende Formulierung ist.

4. Eine Zusammensetzung nach Anspruch 1 oder Anspruch 2, welche eine Lidschatten-Formulierung ist.

**Revendications**

1. Composition cosmétique colorée en poudre appropriée pour l'application locale à la peau humaine, comprenant :

(i) de 0,001 à 30% en poids d'un nitrure de bore en poudre ultrafin ayant une granulométrie de 1 à 10 μm ;
(ii) de 0,001 à 30% en poids d'une matière d'addition cosmétique comprenant un nylon en poudre ayant une granulométrie moyenne se situant entre 2 et 6 μm ;
(iii) de 0,5 à 99% en poids d'un véhicule pharmaceutiquement acceptable choisi dans le groupe consistant en talc, pigments minéraux, oxydes et hydroxydes métalliques, mica, pigments perlés, pigments organiques, silicates minéraux, matières poreuses, carbones, métaux, biopolymères et leurs combinaisons.

2. Composition selon la revendication 1, dans laquelle le nylon est le nylon 12.

3. Composition selon la revendication 1 ou 2, qui est une formulation de fard à joues.

4. Composition selon la revendication 1 ou 2, qui est une formulation de fard à paupières.